# EUROPEAN PATENT APPLICATION

(11) **EP 1 946 725 A1**
(43) Date of publication of application: **23.07.2008**
(21) Application number: 07253916.6
(22) Date of filing: 03.10.2007
(51) Int. Cl.: A61F 2/84

(54) **System for the controlled delivery of stents and grafts**

(30) Priority: 17.01.2007 US 654193
(71) Applicant: Aga Medical Corporation, Plymouth MN 55442-2204 (US)
(72) Inventor: Amplatz, Kurt, St. Paul, Minnesota 55127 (US); Oslund, John C., Blaine, Minnesota 55449 (US); Russo, Patrick, Vadnais Heights, Minnesota 55127 (US)
(74) Representative: Charig, Raymond Julian

(57) **Abstract**

The present invention provides a delivery mechanism for percutaneously routing a stent or graft through the vascular system and procedures for addressing an aneurysm or an otherwise damaged vessel. The delivery system includes an outer tubular guide catheter 20, an inner tubular delivery (pusher) catheter 14 coaxially disposed and slidable relative to the outer guide catheter and an elongated flexible wire or cable 26 that is coaxially insertable through the lumen of the inner tubular catheter and that has a frusto-conical bead affixed at the distal end thereof which is sized to at least partially fit within the lumen of the inner pusher catheter when a proximally directed tension force is applied between the elongated flexible wire or cable 26 with respect to the pusher catheter 14. By inserting a compressed coil spring between a proximal end portion of the pusher catheter 14 and the proximal end portion of the cable 26, the requisite clamping force is maintained to secure the stent or graft to the distal end of the pusher catheter until the compression spring force is removed. With the stent or graft clamped to the distal end of the inner pusher catheter, it can be drawn within the lumen of the outer guide catheter for delivery therewith to the target site.

## Description

### BACKGROUND OF THE INVENTION

### I. Cross-Reference to Related Application:

The present application is a continuation-in-part of co-pending application Serial No. 11/121,386, filed May 4, 2005, the entirety of which is incorporated herein by reference for any purpose.

### II. Field of the Invention:

This invention relates generally to percutaneous transluminal vascular procedures, and more particularly to delivery apparatus for placing a stent, a stent graft or a tubular graft at a desired target location within a subject's vascular system.

### III. Discussion of the Prior Art :

In the field of interventional cardiology, it is now becoming routine to treat stenotic lesions in the vascular system using balloon angioplasty to render more patent a partially occluded blood vessel and to attempt to thwart restenosis by placement of a stent at the site of the treated lesion.

Stents used in these procedures must be capable of assuming a reduced diameter configuration for delivery through a guide catheter or arterial sheath, but which is either self-expanding or "balloon expandable".

In carrying out a balloon angioplasty procedure with stenting, the Seldinger technique is frequently used to gain access to the vascular system, and a tubular introducer having a hemostatic valve for preventing blood loss is inserted through the puncture wound from the skin into the artery. In order to perform the procedure via percutaneous access without a surgical cut-down to expose the femoral artery, an introducer sheath smaller than 14 Fr is required in most patients. The smaller the introducer sheath, the less trauma to the tissue and the easier it is to place and to close the arterial puncture after the procedure. In some cases, a long arterial sheath substitutes a short vascular access sheath and provides a guiding path for delivery of devices to a site proximal the target treatment location. In other cases, a guide catheter is inserted through the introducer sheath and is routed through the vascular system until the distal end portion of the guide catheter is disposed at the ostium of a selected artery having the stenotic lesion.

Next, a catheter may be advanced over a guidewire through the sheath or guide catheter, through the artery to the target treatment site. The catheter may be a balloon catheter, with or without a balloon expandable stent mounted over the balloon, or may be a delivery catheter for a self-expanding stent. Treatment typically involves dilation of the stenotic lesion, followed by placement of a stent at the lesion site. Upon inflation of the balloon, the stenotic region of the artery having a restriction to flow is expanded in the diameter to restore normal blood flow through the arterial segment. A balloon-expandable or self-expanding stent may next be placed in the dilated lesion site to maintain the vessel wall in the expanded diameter state. Balloon expandable stents are placed by inflating a balloon having a stent mounted thereon at the lesion site. Self-expanding stents are typically placed by pulling back a sheath covering a compressed stent mounted at the distal end of the catheter. Following self-expansion of the stent, a balloon dilatation may optionally be used to seat the stent and ensure full expansion. Following the treatment, the catheter, guide wire sheath, etc. are removed from the body and the vascular access site is sealed by compression or other sealing means available.

Stents intended for use in percutaneous transluminal angioplasty applications come in various lengths and diameters to generally approximate the lesion length and normal range of vessel inside diameters at the various treatment sites throughout the body.

Grafts are used for the treatment of aneurysms (an enlargement of the vessel due to a weakened wall) or other vessel abnormalities, and commonly involve a tubular scaffold of metal (typically Nitinol), a polymer or a combination thereof having a fabric (typically polyester) covering designed to prevent blood leakage there through. The grafts are placed to bridge the weakened vascular wall area and provide a new structure to prevent the rupture of the vessel. The grafts are intended, with vascular tissue growth upon the graft surface, to seal at both ends to the vessel wall and throughout the length of the graft to isolate the weakened vessel wall from exposure to the arterial blood pressure which may cause rupture. Treatment sites are commonly in large vessels, such as the abdominal aorta (Abdominal Aortic Aneurysm AAA), and often extend into the iliac arteries, but vascular grafts may be placed in any vessel in the body. The grafts are sized to generally match the native artery size in the treatment region. This construction method for grafts, involving multiple material layers having a large combined wall thickness, is such that the graft, when compressed for delivery, requires a large diameter delivery catheter and thus a large introducer, sheath or guide catheter. The large diameter bulky grafts are also more difficult to navigate through the vasculature and may be more traumatic to the vasculature due to their stiffness. Most prior art vascular grafts of the covered scaffold variety require a 24 Fr (3 French = 1mm) introducer/delivery sheath. In some cases, a cut down through the skin to expose the artery is required, due to the sheath diameter being too large to access the artery in the normal manner using the Seldinger technique. As such, the medical team requires a surgeon to perform a cut-down procedure.

Another issue common to typical vascular grafts is the inability to retrieve or reposition the graft once the graft has been partially deployed. Vascular grafts generally have a Nitinol self-expanding framework and are delivered much like self-expanding stents by a catheter with a pull-back sheath over the compressed graft. A common issue with large stents and grafts is that the force of self-expansion acting against the sheath upon initial sheath pulls back for deployment causes the catheter to jump axially and the stent or graft to be misplaced from the intended target site. The ability to reposition the graft would be beneficial to the treatment.

What is needed, then, is an improved apparatus that provides controlled delivery of self-expanding stents, stent grafts and grafts using percutaneous translumenal catheter delivery. Further, a need exists for a delivery system for stents, stent grafts and grafts wherein the device to be delivered remains affixed to the delivery device, thus allowing the stent, stent graft or graft to be extended from and retracted into a delivery sheath repeatedly until such device is precisely positioned and deemed to be of the appropriate size to address the particular lesion or aneurysm involved. As used herein, a stent is a tubular scaffold for bridging a stenotic lesion in a blood vessel; a stent graft is a stent having a fabric, blood impervious covering; and a graft is a scaffold for bridging a true aneurysm, a false aneurysm or a berry aneurysm. Such devices are collectively referred to herein as a vascular prosthesis or simply prosthesis.

### SUMMARY OF THE INVENTION

The foregoing desired objects are achieved in accordance with the present invention by providing an apparatus for percutaneously delivering a self-expanding stent, stent graft or graft to a target site within a patient's vascular system. The apparatus comprises an outer tubular catheter having a proximal end, a distal end and a lumen extending there between along with an inner tubular pusher catheter also having a proximal end, a distal end and a lumen and where the inner pusher catheter has an outer diameter sized to slidingly fit within the lumen of the outer tubular catheter. An elongate, flexible member is coaxially inserted through the lumen of the inner pusher catheter and has a first bead member affixed to its distal end where the bead is sized to at least partially fit within the lumen of the inner pusher catheter at the distal end of the pusher catheter when a proximally directed tension force is applied to the proximal end of the elongated flexible member with respect to the inner pusher catheter. The maximum diameter of the bead member is sized to sliding fit within the lumen of the outer tubular member. The elongate flexible member may have an optional lumen at least partially there through sized for passage of a guidewire in a sliding manner. Completing the apparatus is a compression spring that is operatively coupled between the proximal end of the inner pusher catheter and a clamp member that is affixed to the elongate member near the proximal end of the elongate member.

The stent, stent graft or graft deployed using the apparatus of the present invention, in one preferred embodiment, comprises a large plurality of very fine braided metal strands exhibiting a self-expanding memory property and which is radially collapsible to a relatively small size for passage through the outer tubular guide catheter but which, when released from the outer tubular catheter, self-expands to a predetermined relatively larger diameter. The number of strands, the diameter of each strand, the pitch and pick of the braid are such that the pore size of the resulting tubular graft is sufficiently small that fibrin present in the blood will close such pores, rendering the graft leak-proof and serving as a platform for vascular tissue in growth including endothelial cells. The braided tubular graft is installed on the delivery system by capturing the free ends of the strands comprising the braided graft at its proximal end between the bead member affixed to the elongate flexible member and the wall defining the lumen of the inner tubular pusher catheter at its distal end. The compression spring is used to maintain the requisite tension force on the elongate member to maintain the ends of the strands pinched between the bead member and the wall of the inner tubular pusher catheter proximate its distal end during delivery and retraction of the prosthesis.

### DESCRIPTION OF THE DRAWINGS

The foregoing features, objects and advantages of the invention will become apparent to those skilled in the art from the following detailed description of a preferred embodiment, especially when considered in conjunction with the accompanying drawings in which like numerals in the several views refer to corresponding parts:
Fig. 1 is a partial side elevation view illustrating the percutaneous delivery system for stents, stent/grafts and grafts configured in accordance with the present invention;
Fig. 2 is a greatly enlarged view of the distal end portion of the assembly of Fig. 1 showing the proximal ends of the wires comprising the braided stent, stent/graft or graft captured at the distal end of the delivery catheter;
Fig. 3 is a view like that of Fig. 2 showing the stent or graft released from the distal end of the delivery catheter;
Fig. 4 is a partial, sectioned view of the distal end portion of an alternative embodiment;
Fig. 5 is a partial, sectioned view of the distal end portion of a further alternative embodiment; and
Fig. 6 is a partial, sectioned view of the distal end portion of a still further alternative embodiment.

### DESCRIPTION OF THE PREFERRED EMBODIMENT

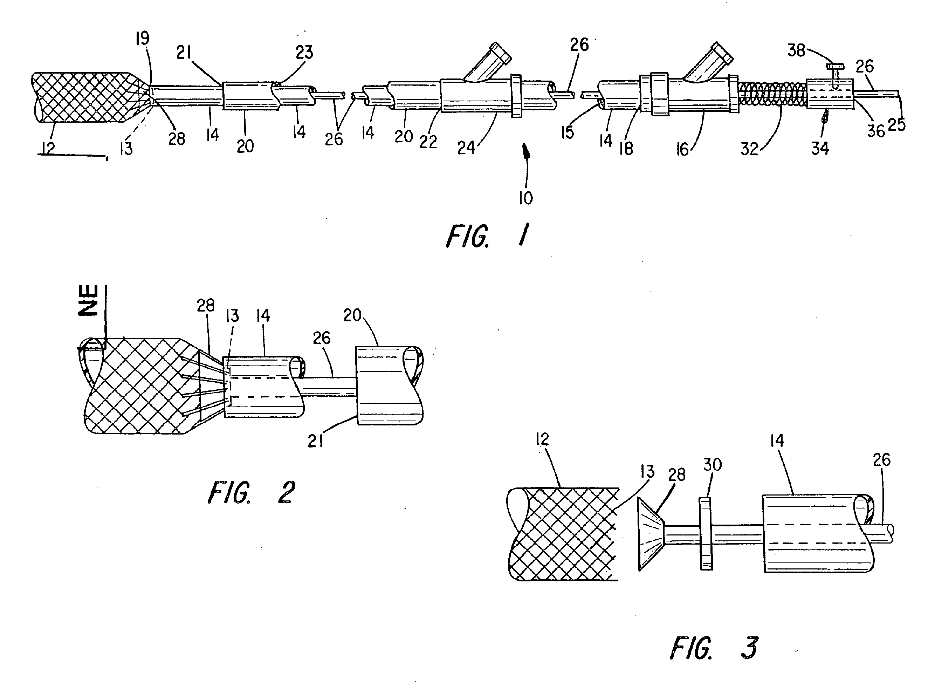

Referring first to Fig. 1, the percutaneous translumenal stent, stent graft or graft delivery system is identified generally by numeral 10 and, as already indicated, is used to deliver a prosthesis 12 to a target site within the vascular system, such as at the location of an abdominal aortic aneurysm for the purpose of exclusion of the aneurysm to prevent further bulging and possible rupture thereof or at the location of a stenotic lesion for vessel patency.

The vascular prosthesis 12 is preferably formed of a metal fabric exhibiting a self-expanded configuration and a collapsed configuration. The prosthesis, when collapsed by longitudinal stretching, can be deployed through the lumen of a catheter and, upon exiting the distal end of the catheter at a target site in a patient's vascular system, will substantially return to its expanded configuration.

As is described in U.S. Patent 5,725,552 to Curtis Amplatz, the metal fabric comprising the prosthesis may comprise a plurality of braided metal strands where the metal is preferably a shape memory alloy such as Nitinol®. In accordance with the present invention, the metal fabric is braided in the form of a tube that can be fitted onto a cylindrical mandrel and then heat-treated so that, in its expanded configuration, the prosthesis will have an internal diameter substantially equal to the outer diameter of the mandrel on which it is heat-treated.

Without limitation, the graft may comprise a 36, a 72, a 144 or a 288 strand tubular wire braid using wires of selected diameters dependent on the number of wires employed in the braiding process Using a tubular braid ranging from 6-40 mm in diameter with a predetermined pitch and pick such that the graft exhibits a pore size less than 100 microns, the graft can be longitudinally stretched to a reduced diameter permitting it to be passed through the lumen of sheaths ranging in size from 6-14 French that can readily be inserted into the vascular system using the Seldinger technique. Upon exit from the distal end of the delivery catheter at the desired target site, the graft 12 will self-expand to a limit defined by the vessel wall in which it is disposed. Post dilatation of the graft can be performed as necessary to obtain full apposition.

Using a metal fabric braided from 36-288 strands or wires whose diameters may be about .001-.010 inch yields a fabric that is rather blood-impervious and within a relatively short time following placement becomes endothelialized. Blood trapped between the outer surface of the graft and the bulge comprising the aneurysm rapidly clots to fill the bulge space with a congealed mass. The lumen of the graft, however, remains patent, allowing continuous blood flow through the treated area of the blood vessel. Examples of embodiments of prosthesis 12, without limitation, are described in detail in a currently filed co-pending patent application entitled "Intravascular Deliverable Stent for Reinforcement of Abdominal Aortic Aneurysm", hereby incorporated by reference.

Those skilled in the art interested in obtaining more information concerning the fabrication of occluding devices using braided structures of the type contemplated herein for the prosthesis 12 are referred to the following patents, each of which is assigned to AGA Medical Corporation, the assignee of the present application, the teachings of which are hereby incorporated by reference.
5,725,552
5,944,738
6,468,303
6,402,772
6,468,301
6,368,339
6,506,204

Turning next to the construction of the first embodiment of the device, the prosthesis delivery apparatus 10 comprises:
an outer tubular catheter 20 (also called a "guiding catheter"), having a distal end 21, a proximal end 22 and a lumen 23, extending there between with a Y adapter 24 on the proximal end in communication with the lumen;
an inner tubular pusher catheter 14, also having a proximal end 18, a distal end 19, and a lumen 15, extending there between, and having an outer diameter sized to slidingly fit within the lumen of the outer tubular catheter 20 with a Y adapter 16 mounted to the proximal end in communication with the lumen;
an elongate, flexible member 26, coaxially inserted through the lumen of the inner pusher catheter 14 and having a first bead member 28, and a washer like member 30, affixed to its distal end where the bead is sized to at least partially fit within the lumen of the inner pusher catheter at the distal end, and the washer 30, is sized to slidingly fit within the lumen 15;
a compression spring 32 operatively coupled between the proximal end of the inner pusher catheter 14 and a clamp member 34, affixed to the elongate member 26, near the proximal end 25,of the elongate member 26; and
prosthesis 12, preferably a self expanding, braided multi- layered Nitinol assembly.

The device 10 for various applications may have a overall length ranging from 30-120 cm to reach arterial treatment sites ranging from iliacs to the aorta to the carotid arteries, via a femoral artery access, and may have an outer tubular catheter diameter of from about 6 to 14 Fr depending on the diameter of the graft needed to treat the respective vessel.

For simplicity, the following detail description, dimensions and materials will be directed to a preferred embodiment intended for the delivery of prosthesis for treatment of an Abdominal Aortic Aneurysm (AAA). Although similar construction and materials may be used for other applications, such as treatment of stenotic lesions in various locations of the body, specific dimensions of the device would be different for each application, due to distance of the treatment site from the access site into the vasculature and size of the vessel being treated, tortuousity and variation in prosthesis size.

The outer catheter 20 may be a long introducer sheath, a guide catheter or steerable delivery sheath having a lumen of a size to receive the inner pusher catheter 14 there through with a sliding fit. Affixed to its proximal end 22 of the outer catheter 20 is a female Luer Y adapter fitting 24. The Y adapter has both a side port and an end port in fluid communication with the lumen. The end port is sized to allow passage of the inner pusher catheter 14 and compressed prosthesis 12. The end port may optionally have a valve to control bleeding from the lumen. The side port may be used to flush the catheter with saline to remove air in preparation for use. The length of the outer catheter 20 is less than the length of the inner pusher catheter 14. The outer tubular member may be constructed by extruding over a copper wire, a 0.001-0.005, preferably 0.003 inch wall thickness tube of PTFE. Winding a 0.002 in. stainless steel braid over the PTFE and extruding over the braid or heat forming a layer of pebax having a wall thickness of 0.005-.008 inch thickness for a combined wall of 0.010 - 0.014 inch. Such techniques are well known to those skilled in the art of sheath or guide catheter construction. This construction provides for a heat shapeable tip and torque control for steering if needed. Additionally a platinum marker band of 0.002-0.003 inch wall thickness may be incorporated into the distal tip for fluoroscopic imaging of the distal tip of catheter 20 as is well known in the art. Alternatively a soft tip containing radiopaque material may be blended and formed into a tip attached to the distal end of the catheter 20.

In the preferred AAA embodiment, the outer catheter has an ID of 0.156 inches and an OD of 0.182 inches and a length of 0.80 cm. It is also contemplated that the outer tubular member 20 may be an appropriately sized, commercially available, long sheath, steerable sheath or guide catheter available for delivery of a variety of catheters.

The inner pushable catheter 14 may be fabricated similar to the construction of the outer catheter or may be a single or multi-layer polymer extruded tube, but is preferably made from a single wall tube extruded from medium density polyethylene. The inner catheter alternatively could also be made from polyethylene, pebax, polyurethane, nylon, polyimide or other materials known in the catheter art. In some applications, it may be possible to fabricate at least a portion of the inner catheter shaft from stainless steel or Nitinol tubing. The inner pusher catheter also has a female Luer Y adapter 16, similar in function and use as described for the outer catheter 20.

In the preferred AAA embodiment, the inner pushable catheter 14 may have an ID of about 0.050 inches and an OD of 0.100 inches and a length of 100 cm.

Elongate flexible member 26 disposed within the lumen of the inner pusher catheter 14 may be made from a wire or cable. Preferably, member 26 is a cable made of stainless steel or Nitinol with a length of 120 cm and a diameter of about 0.030 inches. Attached to the distal end of member 26 by laser welding or other joining means, such as adhesives or a crimp, is a bead 28 that may be spherical, elliptical, "football" shaped or frusto-conically shaped as illustrated in Figure 3. The bead is preferably made from stainless steel (alternatively may be a polymer) and has a length from 0.075 to 0.125 inches, preferably about 0.100 inches. The bead maximum diameter may be larger than the inner pusher catheter lumen but is smaller than the outer catheter lumen. Preferably, the bead has an oval shape with a range of diameter from 0.110 to 0.140 inch preferably a maximum diameter of 0.140 inch and a through center line hole 0.035 inch to fit over the member 26 prior to welding.

Located a short predetermined distance, i.e., approximately 2-20 mm proximal of the bead 28, is a annular washer-like member 30 that is also welded or otherwise fixedly attached to the cable or wire member 26. The washer member 30 (preferably stainless steel), which could be made in many shapes, is designed to fill the cross-sectional space between the outer diameter of the cable and the inner diameter of the inner pusher catheter 14. The washer is sized for a sliding fit within the inner pusher catheter but also sized large enough to serve as a positioning backstop for installing the stent or graft for clamping without allowing space between the washer and inner lumen sufficient to fit the prosthesis. The washer-like member 30 typically has a diameter of about 0.140 inch and a thickness greater than or equal to the diameter and a center hole 0.035 inch diameter to fit over the member 26 prior to welding.

The elongate flexible member 26 must be minimally longer than the inner pusher catheter 14 to allow the elongate flexible member 26 to be extended distally, relative to the inner pusher catheter distal end to totally expose and release the prosthesis extending from the distal end of the inner pushable catheter and to extend proximally from catheter 14 through spring 32 and clamp 34.

Compression spring 32 is preferably made from stainless steel or plated spring steel and sized with an inside diameter to fit over the elongate flexible member 26. The sleeve 36 is provided with a lumen to slide over the member 26 and has a transversely aligned threaded hole in the side wall to mate with the threads of thumbscrew 38. The thumbscrew engages the member 26 to lock the sleeve 36 in place to maintain the desired compressed spring force to retain the prosthesis.

The spring has an outside diameter sized to fit against the proximal end of the Y adapter 16 and against the sleeve 36 on the proximal end. The spring has a length of approximately 1-2 inches and can be compressed between the Y adapter and adjusted clamp 34 with thumbscrew 38 to produce a force sufficient to retain prosthesis 12 between bead 28 and the distal end of lumen 15 of inner catheter 14. This retention force may be in the range of from about ½ to 4 lbs. and preferably in the range 1.25 to 2.0 lbs. Optionally, the sleeve 36 may be permanently fastened to the elongate member in a predetermined position that ensures adequate spring clamping force to retain the prosthesis. In either case the prosthesis may be released by pushing the sleeve 36 distally relative to the Y adapter 16, relieving the clamping force, after the stent is substantially deployed in the target location.

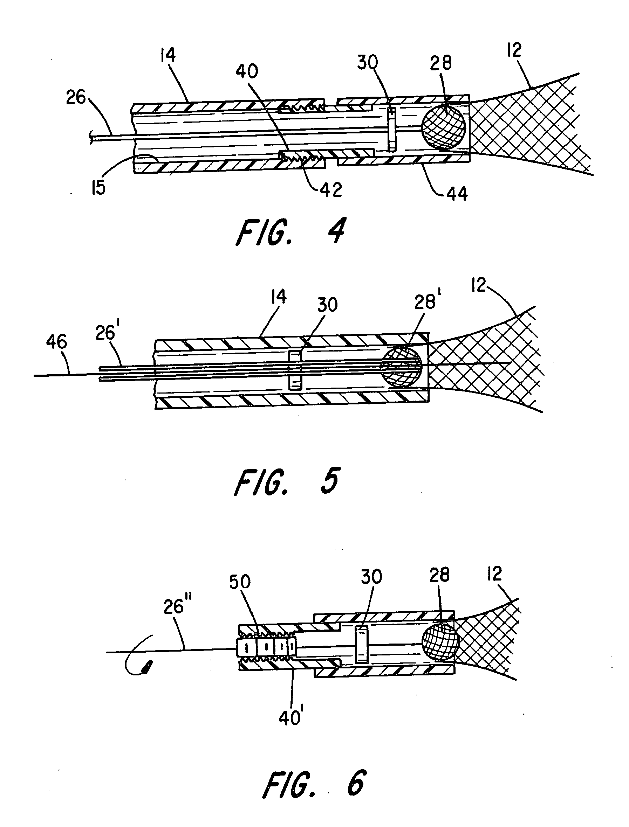

In the preferred embodiment of Figure 4, a rigid, hollow tube 40 may be attached to the distal end of the inner pusher catheter 14. More particularly, a thin walled stainless steel bridge tube 40 is sized to be partially placed inside the distal end of lumen 15 of catheter 14. The bridge tube has a few outside diameter grooves 42 in the proximal section to allow heat forming and fusion of the distal inner pusher catheter to the bridge tube. The distal end of the bridge tube 40 has an outside diameter sized to fit partially within the inside diameter of a further rigid hollow tube 44. The hollow tube 44 is welded by laser to the bridge tube 40. The distal end of the bridge tube 40 serves also as a proximal stop for member 30. The hollow tube 44 is designed to provide a tight fit for the end wires of the braided prosthesis 12 when they are sandwich compressed by spring 32, between bead 28 and the distal end of the hollow tube 44. A tight fit and adequate spring tension are important to retain the graft until the time of desired release. A rigid hollow tube material will prevent creep due to stresses created when the spring 32 compresses the bead 28, against the prosthesis end wires and the distal end of the hollow tube 44. Optionally the distal end of hollow tube 44 or distal end 19 in Fig. 4 on inner tubular member 14 in the embodiment of Fig. 1 may be chamfered proximally or shaped to match the contact surface of bead 28 to improve clamping. Optionally, either or both surfaces of the bead 28 and mating clamp surfaces of inner pusher member distal end may be roughened or coated with a material, such as a polymer, to improve frictional gripping of the graft or stent. Additionally, the bead 28 may have small projections (not shown) extending radially outward on the clamping surface to fit within the openings in the braided prosthesis to further improve clamping.

In the preferred embodiment of Fig. 4, the hollow tube 44 may have an outside diameter of 0.150 inch, a wall of 0.005 inch thickness and a length of 0.75 inches. The bridge tube 40 may have a length of 0.5 inches and an outside diameter of 0.10 inches and a wall thickness of 0.025 inches. There are 2-4 recesses 42 in the proximal outside surface of tube 40 and are approximately 0.030 wide by 0.020 inch deep. The bonding of the pusher catheter 142 to the bridge tube 40 is by reflowing the ID of the pusher catheter shaft into the recesses to create a mechanical lock.

The prosthesis 12 may take many forms such as a self expanding stent, a graft comprised of a self-expanding stent combined with a woven or braided polyester fabric or as in this preferred embodiment, a prosthesis which is fabricated by braiding of Nitinol wires into a tubular inner structure having the main structural characteristics of the prosthesis and surrounding the tubular inner structure at least one or more layers of smaller and more numerous Nitinol braided wires forming tubular structures having smaller openings than those in the structural inner layer. The individual layers of the composite prosthesis being of similar pitch and having similar expansion characteristics and being at least held together by stitching of suture or radiopaque wire. The specific construction and dimension details pertaining to this preferred embodiment prosthesis 12 are described in a co-pending patent application filed concurrently herewith and entitled "Intravascular Deliverable Stent for Reinforcement of Abdominal Aortic Aneurysm", which is hereby incorporated in entirety by reference. For general use purposes of the prosthesis delivery device 10 of this invention, the primary prosthesis characteristic functionally important for compatibility are self-expansion, adequate axial pushability in the collapsed for delivery state and sufficient material on the proximal end to be clamped between the bead 28 and the inner pusher catheter 14 distal end lumen of Fig. 1 or the distal end of the tube 44 in the embodiment of Fig. 4.

To ready the delivery system of Fig. 1 for prosthesis loading, elongate flexible member 26 is located in the inner pusher catheter 14 such that the bead 28 is just distal to the distal end of catheter 14 and the washer-like member 30 distal surface is just proximal the distal end of the inner pusher catheter 14. Next, the free ends of the strands comprising the braid of prosthesis 12, at the proximal end 13, are fed into the lumen of the pusher catheter 14 against the washer like member 30 and are captured between the outer surface of the clamp member 28 and the distal end 15 of the inner pusher catheter 14 and retained by spring 32, as best seen in the greatly enlarged partial view of Fig. 2. The proximal end of the prosthesis must be contained between the distal end of the bead 28 and the annular washer 30 to allow for eventual deployment of the prosthesis. The distal bead 28 securely holds the graft until it is ready to be deployed. The annular washer 3 0 functions to push the braided ends out of the pusher catheter 14 to fully deploy and release the graft or, in the case of proximal withdrawal of the outer catheter 20. To release the stent, washer-like member 30 serves as a backstop to prevent proximal movement of the stent during deployment.

To facilitate loading the prosthesis 12, its expanded configuration is compressed into an optional tear away funnel (not shown), slipped over the tapered bead member 28 into the distal end lumen of catheter 14, positioned against washer like member 30 and the proximal end of the cable or wire member 26 is pulled proximately through catheter 14 (spring 32 assisting) drawing the proximal end of the prosthesis into the inside diameter of catheter 14. So long as the spring tension is maintained, the free ends 13 of the braided prosthesis 12 will remain captured.

As shown in Fig. 1, clamp 34 is positioned on elongate flexible member 26 to compress the spring 32 to a prescribed force suitable for prosthesis retention and then thumbscrew 38 is tightened to maintain the clamp 34 in position. Alternatively, sleeve 36 is crimped or otherwise affixed to member 26 in a prescribed position without a thumbscrew needed.

Prior to the prosthesis loading procedure, an outer catheter loading sleeve 20 is advanced over the distal end of the inner pusher catheter 14. This sleeve is preferably made of PTFE and is sized to a close but sliding fit over catheter 14 and has a slit or a spiral cut along the full axial length or may be optionally a tear-away sleeve. The wall thickness of the sleeve is selected to be sufficient to structurally resist diameter expansion by the prosthesis. The sleeve is longer than the prosthesis length. After the prosthesis is clamped in place, as previously described to catheter 14, the outer catheter loading sleeve 20 is advanced distally over the prosthesis to compress the prosthesis to a diameter that will later facilitate loading into the proximal end of the outer tubular catheter 20. A funnel (not shown) used to help load the proximal end of the prosthesis during loading may be used to facilitate the loading sleeve advancement by moving both distally together to help compress the prosthesis to the sleeve inside diameter. This loading sleeve may also serve as a shipping protection for the prosthesis.

During a procedure, the physician gains access to the femoral artery by the Seldinger technique. The physician may choose to use either a long sheath or steerable sheath as the outer tubular catheter 20 or use a short access sheath with a guide catheter or secondary sheath as the outer tubular catheter 20. In either case, a long guidewire (not shown), typically .038" in diameter by up to 150 cm long, is placed in the femoral artery through the access. The outer tubular catheter 20 (as selected) is advanced over the guidewire through the vasculature to the target treatment site (across an aneurysm or across a stenotic lesion) such that the distal tip of the catheter 14 is just proximal the location desired for placement of the distal end of the prosthesis. The inside diameter of the catheter 14 is selected to match the inner pusher catheter 14 outside diameter such that catheter 14 may fit slidingly within the lumen of catheter 20.

Once catheter 20 is positioned as desired, the guidewire is removed. Next, the distal end of the outer catheter loading sleeve is placed into the inlet to the lumen at the proximal end of the outer tubular catheter 20 through Y adapter 24. The inner pusher catheter 14 with compressed prosthesis 12 clamped in the distal end and the distal portion of the prosthesis enclosed within the loading sleeve may now be slidingly advanced into the catheter 20 while holding back the sleeve, until the prosthesis and distal portion of the inner pusher catheter 14 are now within the lumen of the outer catheter 20. This forward advancement of the stent is possible within the lumen of the outer catheter because the compressed diameter of the braided prosthesis aligns the helical wires into a more axially aligned orientation making the prosthesis very pushable. At this point, the sleeve may be removed depending on the design type by placing the catheter into the sleeve slit and pulling the sleeve off, or if a tear away design, pulling a tear strip and removing the sleeve through the slit.

The inner pusher catheter 14 and prosthesis 12 are advanced distally together until the distal tip of the prosthesis is at the distal end of the outer tubular catheter 20. This can be visualized by fluoroscopy as the outer catheter 20 has a radiopaque platinum marker band embedded in the distal tip and the prosthesis is sufficiently radiopaque. Alternatively, the positioning can be obtained by aligning marks on the catheters 14 and 20 placed for this purpose by design on the catheter shaft in the proximal portion outside the body. The prosthesis preferably has radiopaque markers placed by platinum stitching at the midpoint of the axial length. The outer catheter and inner pusher catheter therein are now advanced or retracted together to place the prosthesis midpoint markers at the center of the lesion or aneurysm.

The prosthesis may now be deployed by holding the outer catheter 20 and advancing the inner pusher catheter 14 a few mm, e.g., 1-5 mm, out the end of the catheter 20 distal tip. While holding back the inner catheter 14, the outer catheter 20 may now be pulled back proximally until the distal end of catheter 20 lines up (using fluoroscopy) with the midpoint markers on the prosthesis 12. The physician now determines if the prosthesis is positioned as desired or not. If the position of the prosthesis is as desired, the outer catheter 20 may be further retracted relative to the inner catheter 14 such that the distal tip of catheter 20 is just proximal the distal end of the inner catheter distal. The prosthesis may now be released by holding the inner pusher catheter 14 in place while pushing the sleeve 36 or clamp 34 or member 26 distally to release the bead 28 from clamping the prosthesis and by placing the bead distally from the distal end of pusher catheter 14 and then, proximally moving the inner catheter 14 to release the prosthesis proximal wire ends from the lumen of catheter 14. The prosthesis 12 is now fully deployed.

The inner catheter 14 is now removed from the lumen of outer catheter 20. If it is desired to balloon dilate the prosthesis, a balloon catheter and suitable guidewire may be advanced through the outer catheter 20. The outer catheter may be removed after all catheter work is completed along with a short access sheath, if used, and the arterial puncture sealed by compression or arterial sealing device as well know in medical practice and in common use.

The delivery system of the present invention allows the prosthesis to be repositioned as long as the prosthesis has not been released from the bead clamp or the elongate flexible member 26 is not advanced distally beyond the point where the proximal prosthesis wire ends are not within the inner pushable catheter lumen. The prosthesis can be repositioned into outer catheter 20 by holding the position of inner pusher catheter 14 and advancing the outer catheter 20 distally over the partially deployed prosthesis. The catheters may then be repositioned for a second deployment attempt. A partially deployed prosthesis may be dragged proximally a small distance by proximal movement of the inner catheter 14, if desired, without advancing the outer catheter back over the prosthesis, but it is better not to do this to reduce trauma to the artery. If the prosthesis proves the wrong size during partial deployment, the prosthesis may be recovered into the outer catheter and removed from the body.

In an alternative embodiment shown in Fig. 5, the elongate flexible member 26 is a tubular structure rather than a cable or wire and having a lumen there through sized for a sliding fit to a guidewire 46 coaxially therein. The member 26 may be fabricated by extruding a thin-walled high-density polyethylene tubing (HDPE) and braiding a 0.001" stainless steel wire over the top to add push and stiffness characteristics to the member. A layer of pebax or HDPE may be optionally be added over the braid to lock in the braid. The HDPE inner surface provides a lubricious surface for ease of guidewire tracking. In all other regards, the construction is similar to the first embodiment except the bead 28' has a lumen and is placed over the member 26' by adhesive and the clamp sleeve 36 is also bonded in place. The lumen in the elongate flexible member allows the device to be advanced over a guidewire. This design has advantages, in certain uses, such as in carotid stenting or in locations where it may not be possible or practical to advance an outer catheter to the target site of treatment, such as where the carotid artery ostium at the aorta is at a sharp angle making access difficult for a stiff outer catheter. In such cases, a guide catheter with a special tip shape may be advanced to seat only to the carotid ostium. Alternatively, steerable sheaths may be designed to help gain access, but may not be able to pass fully to the treatment site.

For prosthesis delivery system 10 to function in this situation, a new sheath is required to enclose the prosthesis through the vasculature beyond the end of the guide catheter or steerable sheath. In this second embodiment, a thin walled HDPE extruded outer tubular catheter 20, sized with a lumen to slidably accept the inner pusher catheter 14 therein has a length less than the inner pusher catheter 14 but sufficient to reach from outside the body through the femoral artery access site to a position across the target treatment site. Once the guide catheter or steerable sheath is in place, the inner pusher catheter 14 and prosthesis 12 may be introduced into a thin-walled outer tubular member 20, just as described previously, except that a guidewire, such as a 0.014" coronary wire, is inserted into the lumen of the elongate flexible member 26' and then an outer catheter loading sleeve is inserted into a Y adapter 24 connected to the proximal end on the outer tubular catheter 20. This may be done outside the body. The inner pusher catheter 14 and prosthesis 12 are advanced together until the distal end of the prosthesis is adjacent the distal end of the outer catheter 20 with the guide wire extending distally by a few cm. The distal end of the guidewire 46 and the assembly of catheters 14 and 20 will now be loaded into the proximal end of either the guide catheter or steerable sheath until positioned near the distal end of the guide or sheath. From this point forward, the guidewire is advanced and steered independently until it is across the target treatment site. With the wire held in place, the catheter assembly is advanced as a unit until the distal end is just proximal the desired distal position of the prosthesis. Deployment and repositioning is the same as previously described. It is important in this second embodiment that the guide catheter or steerable sheath be sized up to fit over the thin walled outer catheter 20.

In a further alternative embodiment of the prosthesis delivery system 10, similar to the previously described embodiments, a significant difference relates to the bead 28. The bead in this case may be any of the previously shaped beads, but is sized such that the maximum diameter of the bead is less than the inside diameter of the lumen of the distal end region of inner pusher catheter 14. Specifically, the diameter of the bead is sized to compress the end wires of the prosthesis between the bead and the inside diameter of the catheter 14 distal lumen such that the friction that results from this fit is sufficient to retain the prosthesis during advancement or retraction of the inner catheter 14 relative to the outer catheter 20 and during deployment of the prosthesis. Since the retention force is adequate to clamp the prosthesis on its own, the spring 32, sleeve 36 and thumbscrew 38 are no longer needed. This simplifies the catheter 10 and eliminates any need for a adjusting or setting a prescribed spring force. In this latter embodiment, the distal end of inner pusher catheter 14 interfacing with the proximal end of the prosthesis and the bead may be a thin walled stainless steel tubing connected by adhesive to the inner tubing of catheter 14. Use of this short metal tubing section (2-20 mm) allows for precise inside diameter dimensions for control of fit and friction. In addition, it is contemplated that the bead may have a pattern machined, etched or otherwise formed into the outer surface to mate with the braided wire surface to trap the stent between the wall of the bead and the metal tube. The bead may also have a cylindrical shape in addition to those previously described. It is also anticipated that, rather than a recessed shape pattern matching the braid pattern, a smooth surfaced bead could be coated with a conformable material, such as urethane, to improve friction and conform to the surface indications in the braid spacing to help secure the prosthesis.

In a third embodiment of the device 10 illustrated in Fig. 6, the bridge tube 40' has a female thread 50 coaxially placed to engage a corresponding raised male thread on the elongate flexible member 26". When the threads of member 26" are engaged with the threads of bridge tube 40', rotation by a handle (not shown) outside the body connected to the proximal end of member 26" either advances or retracts member 26" and, therefore, bead 28 to clamp or release the hold on the prosthesis 12.

While a preferred embodiment of the present invention has been described, it should be understood that various changes, adaptations and modifications may be made therein without departing from the spirit of the invention and the scope of the appended claims. For example, rather than front loading the pusher catheter 14 carrying the elongate member 26 and the prosthesis 12 by feeding the proximal end of the pusher catheter through the distal end of the delivery sheath 20 and then along the length of the delivery sheath, it is also contemplated that a loader tube containing the prosthesis be coupled to the Luer fitting 24 and the pusher wire 26 be clamped to the prosthesis at the time of use and used to advance the prosthesis down the delivery sheath until it approaches the distal end of the delivery sheath 20.

## Claims

1. Apparatus for percutaneously delivering a self-expanding prosthesis to a target site within a patient's vascular system comprising:
(a) an outer tubular guiding catheter having a proximal end, a distal end and a lumen extending therebetween;
(b) an inner tubular pusher catheter having a proximal end, a distal end and lumen extending therebetween, the inner pusher catheter having an outer diameter sized to slidingly fit within the lumen of the guiding catheter; and
(c) an elongate, flexible member coaxially insertable through the lumen of the inner pusher catheter, said elongate, flexible member having a proximal end and a distal end, said distal end having a first bead member affixed thereto sized to at least partially fit within the lumen of the inner pusher catheter at said distal end of the inner pusher catheter when a proximally directed tension force is applied to the elongate flexible member with respect to the inner pusher catheter.

2. The apparatus as in claim 1 wherein the elongate member further includes a second bead member affixed thereto at a location a predetermined distance proximal of the first bead member, the second bead member sized to slidingly fit within the lumen of the inner tubular catheter.

3. The apparatus as in claim 1 wherein the first bead member is one of spherical, oval and frusto-conically shaped.

4. The apparatus as in claim 3 wherein the first bead member has a friction enhancing surface thereon.

5. The apparatus as in claim 4 wherein the friction enhancing surface is a knurled surface.

6. The apparatus as in claim 4 wherein the friction enhancing surface is a polymer.

7. The apparatus in claim 1 wherein the first bead member is spherical.

8. The apparatus as in claim 2 wherein the second bead member comprises an annulus.

9. The apparatus as in claim 1 wherein a proximal end portion of the self-expanding prosthesis is captured between the first bead member and a wall defining the lumen of the inner pusher catheter at the distal end thereof when the tension force is being applied.

10. The apparatus as in claim 1 wherein the elongate, flexible member comprises a wire.

11. The apparatus as in claim 1 wherein the elongate, flexible member comprises a cable.

12. The apparatus as in claim 1 wherein the elongate, flexible member comprises a tube having a lumen extending the length thereof.

13. The apparatus as in claim 12 and further including a guidewire inserted through the lumen of the elongate, flexible member.

14. The apparatus as in claim 1 wherein the outer tubular guide catheter includes a first Y adapter having a Luer fitting on the proximal end thereof and the inner pusher catheter includes a second Y adapter with a Luer fitting on the proximal end thereof.

15. The apparatus as in claim 14 and further including a compression spring disposed in surrounding relation with respect to the elongate, flexible member between the Luer fitting of the second Y adapter and a clamp member releasably affixed to the elongate, flexible member.

16. The apparatus as in claim 15 wherein the self-expanding prosthesis is released from the distal end of the inner catheter when the first bead member is advanced distally with respect to the inner catheter.

17. The apparatus as in claim 1 wherein the self-expanding prosthesis comprises a plurality of fine metal wire strands interwoven to form a tubular metal fabric having open proximal and distal ends and where the metal strands at the proximal end of the tubular metal fabric are adapted to be captured between the first bead member and a wall defining the lumen of the inner pusher catheter at the distal end thereof.

18. The apparatus as in claim 17 wherein the fine wires comprise shape memory alloy wires.

19. The apparatus as in claim 18 wherein the shape memory alloy is Nitinol.

20. Apparatus for percutaneously delivering a self-expanding prosthesis to a target site within a patient's vascular system comprising:
(a) a tubular inner catheter having a proximal end, a distal end and lumen extending therebetween, the inner catheter having an outer diameter adapted to slidingly fit within the lumen of a guiding catheter; and
(b) an elongate, flexible member coaxially insertable through the lumen of the inner tubular catheter, said elongate, flexible member having a proximal end and a distal end, said distal end having a first bead member affixed thereto sized to at least partially fit within the lumen of the inner tubular catheter at said distal end of the inner tubular catheter when a proximally directed tension force is applied to the elongate flexible member with respect to the inner tubular catheter.

21. The apparatus as in claim 20 and further including a compression spring operatively coupled between the proximal end of the inner tubular catheter and a clamp member releasably affixed to the elongate member.

22. The apparatus as in claim 20 wherein the elongate member further includes a second bead member affixed thereto at a location a predetermined distance proximal of the first bead member, the second bead member sized to slidingly fit within the lumen of the tubular catheter.

23. The apparatus as in claim 22 and further including a stop member disposed within the lumen of the tubular inner catheter a predetermined distance proximal of a distal end of the tubular inner catheter, the stop member adapted to engage the second bead member as the elongated flexible member is slid in a proximal direction within the lumen of the tubular inner catheter.

24. The apparatus as in claim 20 wherein the first bead member is frusto-conically shaped.

25. The apparatus as in claim 20 wherein the first bead member is either spherical or ovaloid.

26. The apparatus as in claim 20 wherein the first bead member has a friction enhancing surface thereon.

27. The apparatus as in claim 26 wherein the friction enhancing surface is a knurled surface.

28. The apparatus as in claim 26 wherein the friction enhancing surface is a polymer.

29. The apparatus as in claim 22 wherein the second bead member comprises an annulus.

30. The apparatus as in claim 21 wherein a proximal end portion of the self-expanding prosthesis is captured between the first bead member and a wall defining the lumen of the inner tubular catheter at the distal end thereof when the tension force is being applied.

31. The apparatus as in claim 20 wherein the elongate, flexible member is a wire.

32. The apparatus as in claim 20 wherein the elongate, flexible member comprises a cable.

33. The apparatus as in claim 21 wherein the compression spring is disposed in surrounding relation with respect to the elongate member between a Luer fitting on the tubular inner catheter and the clamp member.

34. The apparatus as in claim 21 wherein the self-expanding prosthesis is released from the distal end of the inner tubular catheter when the first bead member is advanced distally with respect to the inner catheter.

35. The apparatus as in claim 20 wherein the self-expanding prosthesis comprises a plurality of fine metal wire strands interwoven to form a tubular metal fabric having open proximal and distal ends and where the metal wire strands at the proximal end of the tubular metal fabric are adapted to be captured between the first bead member and a wall defining the lumen of the inner tubular catheter at the distal end thereof when said tension force is applied.

36. The apparatus as in claim 35 wherein the fine wires comprise shape memory alloy wires.

37. The apparatus as in claim 35 wherein the shape memory alloy is Nitinol.

38. The apparatus as in claim 30 and further including a rigid tubular extension affixed to the distal end of the tubular inner catheter.

39. The apparatus as in claim 38 wherein the rigid tubular extension includes a threaded surface on an internal diameter thereof and the elongate flexible member includes a cylinder portion having a threaded surface mating with the threaded surface on the tubular extension such that rotation of the elongate, flexible member translates the first bead member in an axial direction.

40. A method for controllably delivering a braided, self-expanding tubular prosthesis to a selected site in the vascular system comprising the steps of:
(a) providing the combination of:
(i) a flexible tubular inner catheter having a proximal end, a distal end and a lumen extending therebetween, the inner catheter having an outer diameter adapted to slidingly fit within the lumen of a guiding catheter,
(ii) an elongate, flexible member coaxially insertable through the lumen of the inner tubular catheter, said elongate flexible member having a proximal end and a distal end, said distal end having a first bead member affixed thereto sized to at least partially fit within the lumen of the inner tubular catheter at said distal end of the inner tubular catheter when a proximally directed tension force is applied to the elongate flexible member with respect to the inner tubular catheter,
(iii) a braided tubular prosthesis with the proximal ends of individual strands comprising the prosthesis captured between the first bead member and the distal end of the inner tubular catheter;
(b) feeding the inner tubular catheter with the braided tubular prosthesis attached through a lumen of the guiding catheter and out a distal end thereof;
(c) moving the elongate flexible member relative to the tubular inner catheter to release the tubular prosthesis from the distal end of the tubular inner catheter.

41. The method of claim 40 wherein the step of feeding the inner tubular catheter with the braided prosthesis out the distal end of the guiding catheter is achieved either by drawing the guiding catheter proximally while holding the inner tubular catheter and prosthesis fixed or by advancing the inner tubular catheter in the distal direction while holding the guiding catheter fixed.

42. A method for controllably delivering a braided, self-expanding tubular prosthesis to a selected site in the vascular system comprising the steps of:
(a) providing the combination of:
i) an outer guiding catheter;
ii) A flexible, tubular inner catheter having a proximal end, a distal end and a lumen extending therebetween the inner catheter having an outer diameter adapted to slidingly fit within a lumen of the guiding catheter,
iii) an elongate, flexible member coaxially insertable through the lumen of the inner tubular catheter, said elongate flexible member having a proximal end and a distal end, said distal end having a first bead member affixed thereto sized to at least partially fit within the lumen of the inner tubular catheter at said distal end of the inner tubular catheter when a proximally directed tension force is applied to the elongate flexible member with respect to the inner tubular catheter,
(iv) a braided tubular prosthesis with the proximal ends of individual strands comprising the prosthesis captured between the first bead member and the distal end of the inner tubular catheter
(b) routing the guiding catheter through the vascular system until a distal end of the guiding catheter is proximate said selected site;
(c) advancing the tubular inner catheter, elongate flexible member and braided tubular prosthesis through the lumen of the outer guiding catheter until the tubular prosthesis is disposed at the distal end of the guiding catheter;
(d) manipulating the inner tubular catheter relative to the guiding catheter to expose the braided tubular prosthesis out from the distal end of the guiding catheter;
(e) releasing the braided tubular prosthesis from the distal end of the inner tubular catheter when the braided tubular prosthesis is properly located at the selected site or drawing the braided tubular prosthesis back into the guiding catheter when the braided tubular prosthesis is not properly located at the selected site; and
(f) repeating step (e) until the tubular prosthesis is properly located at the treatment site.

43. A method for controllably delivering a braided, self-expanding tubular prosthesis to a selected site in the vascular system comprising the steps of:
(a) providing the combination of:
i) an outer guiding catheter;
ii) an elongated guidewire;
iii) a flexible, tubular inner catheter having a proximal end, a distal end and a lumen extending therebetween the inner catheter having an outer diameter adapted to slidingly fit within a lumen of the guiding catheter,
iv) an elongate, flexible, tubular member coaxially insertable through the lumen of the inner tubular catheter, said elongate flexible member having a proximal end and a distal end and a lumen extending therebetween for receiving the guidewire therethrough, said distal end having a first bead member affixed thereto sized to at least partially fit within the lumen of the inner tubular catheter at said distal end of the inner tubular catheter when a proximally directed tension force is applied to the elongate flexible member with respect to the inner tubular catheter,
(v) a braided tubular prosthesis with the proximal ends of individual strands comprising the prosthesis captured between the first bead member and the distal end of the inner tubular catheter
(b) routing the guiding catheter through the vascular system until a distal end of the guiding catheter is proximate said selected site;
(c) advancing the guidewire through the guiding catheter;
(d) advancing the tubular inner catheter, elongate tubular flexible member and braided tubular prosthesis over the guidewire and through the lumen of the outer guiding catheter until the tubular prosthesis is disposed at the distal end of the guiding catheter;
(e) manipulating the inner tubular catheter relative to the guiding catheter to expose the braided tubular prosthesis out from the distal end of the guiding catheter;
(f) releasing the braided tubular prosthesis from the distal end of the inner tubular catheter when the braided tubular prosthesis is properly located at the selected site or drawing the braided tubular prosthesis back into the guide catheter when the braided tubular prosthesis is not properly located at the selected site;
(g) repeating step (f) until the tubular prosthesis is properly located at the treatment site.
(h) removing the guiding catheter, the inner catheter, the flexible member and the guidewire from the vascular system.
